Europäisches Patentamt

European Patent Office

Office européen des brevets

(11) Publication number: **0 406 003 A1**

# EUROPEAN PATENT APPLICATION

(21) Application number: 90307140.5

(22) Date of filing: 29.06.90

(51) Int. Cl.5: **C12N  15/52**, C12N 15/54, C12N 1/21

The applicant has filed a statement in accordance with Rule 28 (4) EPC (issue of a sample only to an expert). Accession number of the deposit: NCIMB 40153.

(30) Priority: 30.06.89 IE 2131/89

(43) Date of publication of application:
02.01.91 Bulletin  91/01

(84) Designated Contracting States:
AT BE CH DE DK ES FR GB GR IT LI LU NL SE

(71) Applicant: **UNIVERSITY COLLEGE, CORK
College Road
Cork(IE)**

(72) Inventor: **O'Gara, Fergal
Fahan Hilton, Model Farm Road
Cork(IE)**
Inventor: **Condon, Seamus
Doirin Aluinn, Upper Curraheen Road
Bishopstown, Cork(IE)**
Inventor: **Ross, Paul
3 Capwell Road
Cork(IE)**

(74) Representative: **Ruffles, Graham Keith et al
MARKS & CLERK 57-60 Lincoln's Inn
FieldsIds
London WC2A 3LS(GB)**

(54) Marker genes for genetic manipulation.

(57) Marker genes which can be safely substituted for antibiotic resistance marker genes in genetic engineering procedures are described. Such marker genes are thymidylate synthase genes derived from generally-recognised-as-safe microorganisms. Vectors comprising these genes are also described.

## MARKER GENES FOR GENETIC MANIPULATION

The present invention relates to environmentally safe selectable markers for use in genetic manipulation procedures, and in particular to the use of the thymidylate synthase gene as such a marker.

To date the genetic engineering of microorganisms has depended almost entirely on the use of antibiotic resistance genes, either to genetically label recipient cells or to identify and maintain plasmids used as vectors in genetic engineering protocols. The release of genetically engineered microbes (GEMS) into the general environment, their use in agriculture and food processing industries or their use in health care industries is likely to be curtailed by regulatory agencies if the strains carry antibiotic resistance genes. There is an obvious need, therefore, for marker genes which can be substituted for antibiotic resistance genes and which will not have any consequence which might retard clearance by regulatory agencies of GEMS carrying the substitute marker genes.

The necessity for selectable marker genes to substitute for antibiotic resistance genes has been mentioned in the recent literature in relation to the use of genetically engineered microbes for food applications. Herman and McKay (1986) commented on the unsuitability of antibiotic resistance markers and suggested that the gene for B. galactosidase which they cloned from Streptococcus thermophilus was a suitable food grade marker gene. In three separate papers Dutch workers also commented on the unsuitability of antibiotic resistance genes for food applications and suggested substitution by genes associated with lactose metabolism, copper resistance and bacteriocin resistance (de Vos, 1986; de Vos, 1987; de Vos and Simons, 1988).

In addition, in the case of the development of attenuated vaccine strains, particularly those derived from pathogenic microorganisms, it would be highly desirable to carry out genetic manipulation without using antibiotic resistance markers. This would avoid the possibility of creating pathogenic microorganisms bearing antibiotic resistance genes, which would be extremely hazardous if accidentally released into the environment.     .

It is an object of the invention to develop a marker gene which can be safely substituted for antibiotic resistance genes in genetic engineering procedures.

The present inventors have discovered that the thymidylate synthase (TS) gene is suitable to replace antibiotic resistance genes as a selection marker. In particular, the thymidylate synthase gene from Streptococcus lactis , a species of bacteria used routinely for cheese manufacture (and therefore established as a safe microbe) is a suitable candidate as a marker gene which can substitute for antibiotic resistance genes, especially as a "food grade" marker gene. Thymidylate synthase (5, 10-methylenetetrahydrofolate:dUMP C-methyl-transferase; EC 2.1.1.45) plays a key role in DNA synthesis; it catalyses the reductive methylation of dUMP to dTMP with concomitant conversion of the cofactor 5, 10-methylenetetrahydrofolic acid to 7,8-dihydrofolic acid. This activity is an essential step in de novo biosynthesis of DNA. Cells which have lost TS activity, through mutation in the TS gene, cannot make DNA and cannot survive unless supplied with thymine or thymidine, which they convert to dTMP by an alternative pathway.

The thymidylate synthase gene has been extensively studied because of its essential role in synthesis of DNA de novo and because it is an important target enzyme for chemotherapy. The TS gene has been cloned and sequenced from at least 10 sources (Table 1). The deduced amino acid sequences of TS proteins have been compared (Hardy et al ., 1987; Bzik et al ., 1987) and show remarkable homology. However, the present inventors have surprisingly found that it has not previously been suggested that the TS gene could be used as a marker gene to construct vectors or to label cells as a replacement for antibiotic resistance genes.

The TS gene from Lactobacillus casei is another candidate as a suitable antibiotic resistance substitute gene as its source would be regarded as a safe microbe.

According to the present invention there is provided a vector devoid of antibiotic resistance genes and bearing a thymidylate synthase gene as a selection marker.

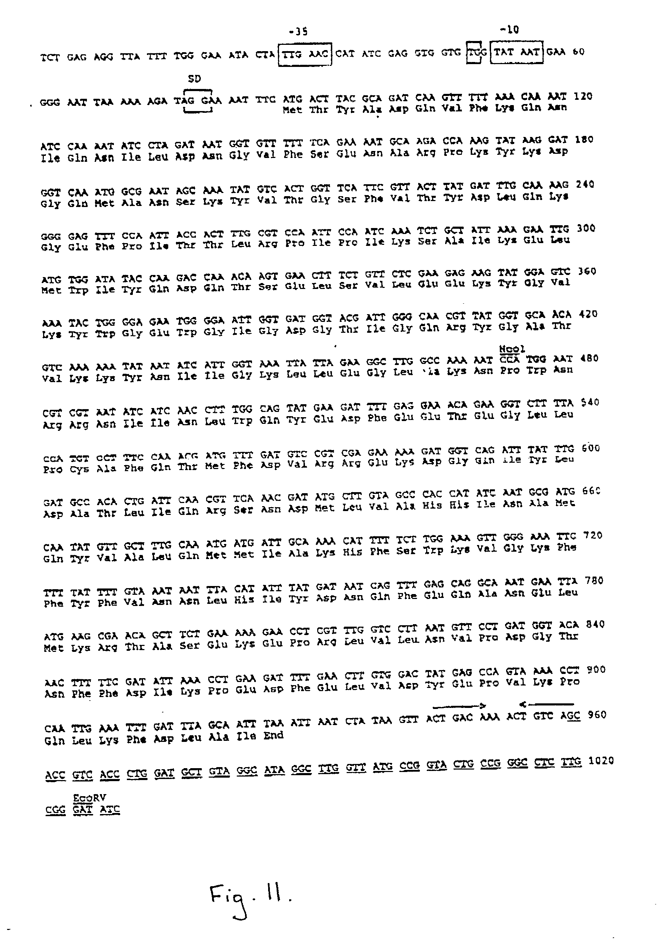

In a particular aspect the vector may bear a thymidylate synthase gene derived from a G.R.A.S. (generally recognised as safe) micro-organism. The thymidylate synthase gene may suitably be derived from Lactobacillus casei or Streptococcus lactis . Particularly preferred are vectors bearing the thymidylate synthase gene deposited in accordance with the Budapest Treaty, in plasmid pGDT11 at the National Collection of Industrial and Marine Bacteria, Torry Research Station, Aberdeen, Scotland, under accession No. 40153 on 2nd June 1989, or the thymidylate synthase gene having the DNA sequence shown in Fig. 11 and vectors derived therefrom, mutants thereof and DNA from any of the foregoing, provided that the essential property of encoding thymidylate synthase activity is retained.

The invention further relates to the thymidylate synthase gene deposited in accordance with the Budapest Treaty, on 2nd June 1989 at the National Collection of Industrial and Marine Bacteria, Torry Research Station, Aberdeen, Scotland, under accession No. 40153, and to the thymidylate synthase gene having the DNA sequence shown in Fig. 11, and genes derived therefrom, mutants thereof and DNA from any of the foregoing, provided that the essential property of encoding thymidylate synthase activity is retained.

The present invention also provides vectors and genes which are substantially similar to· the above vectors and genes, that is vectors and genes which encode thymidylate synthase activity. Preferaby, such vectors or genes comprise mutants of the above described vectors or genes, or which have at least 30%, more preferably 50% and most preferably at least 80% homology with the region of DNA in the vector or gene which encodes thymidylate synthase activity.

"Substantially similar" vectors and genes should encode thymidylate activity.

It will be appreciated that such vectors and genes may vary widely within the scope of the invention and may be derived ab initio or from the deposited material or sequence disclosed herein, provided that the thymidylate synthase activity is retained. The vectors, genes and/or the coding DNA may be in any suitable form which may include mutations such as deletions, insertions, inversions and variants based on the degeneracy of the genetic code and/or replacement of non-essential amino acids.

The invention also provides host cells comprising a vector or a gene as defined above.

The invention relates to all of the above materials in isolated and/or biologically pure form.

In a further aspect the invention provides a method of genetically labelling a cell which has received a gene of interest which comprises coupling the gene of interest with a thymidylate synthase gene as disclosed above and transforming a recipient cell, which is devoid of thymidylate synthase activity, with the coupled gene of interest and thymidylate synthase gene, such that cells which have received the gene of interest will be thymidylate synthase positive.

The invention also provides a method of stably maintaining a gene of interest in a cell which has received the gene of interest by genetic manipulation, comprising coupling the gene of interest to a thymidylate synthase gene, transforming a recipient cell, which is devoid of thymidylate synthase activity, with the coupled gene of interest and thymidylate synthase gene, and maintaining the recipient cell in a culture medium devoid of or limited in thymidine or thymine such that only cells which maintain the thymidylate synthase gene grow.

Strains of microorganism devoid of thymidylate synthase activity (i.e. TS⁻) can easily be distinguished from normal TS⁺ strains. In chemically defined growth media, which support good growth of TS⁺ strains, TS⁻ cells die unless the medium is supplemented with thymine or thymidine. Many complex growth media containing yeast extract, peptones etc. usually do not have sufficient thymine or thymidine to support growth of TS⁻ strains. On plates which support formation of clearly visible colonies by TS⁺ cells, TS⁻ cells fail to grow or at best form a barely visible background growth. For example, TS⁺ Escherichia coli form substantial colonies on Luria and Bertani (LB) plates, LB plates being used routinely for genetic engineering work (Maniatis et al . 1982) whereas TS⁻ cells form clearly visible colonies only when LB is supplemented by thymidine or thymine (up to 25mg/L).

Strains which are TS⁻ can be converted to TS⁺ by transforming the TS⁻ cells with plasmids which have a cloned S. lactis TS gene or a TS gene from other microorganisms. For many applications a safe microorganism would be preferred. The TS⁺ transformants are easily selected by plating on media such as LB which does not support colony formation by the untransformed TS⁻ cells. This means that the S. lactis TS gene functions as a positive selection gene. Plasmids with the S. lactis TS gene can be used as vectors to bring other unselected genes into suitable TS⁻ recipients and such vectors can therefore be developed for genetic engineering work as substitutes for vectors with antibiotic resistance genes.

Furthermore, cloned vector plasmids with the S. lactis TS⁻ gene will be stably maintained in TS cells in media or environments which do not have sufficient thymine or thymidine, as loss of the plasmid results in cell death. In the specific case of the dairy industry milk does not have sufficient thymine or thymidine to support proper growth of TS⁻ E. coli cells whereas the presence of the S. lactis TS plasmids in such cells allows full growth and stable maintenance.

The abreviations TS and Thy are both used to denote the thymidiylate synthase gene in the literature, while in the present specification the abbreviation TS alone is used.

Detailed Description of the Drawings

Fig. 1a : Growth of E. coli X2913 (TS⁻) with plasmid pLAFR1 in the presence (squares) and absence

EP 0 406 003 A1

(triangles) of thymidine.

Fig. 1b : Growth of E. coli X2913 with complementing cosmid, pBT5, in the presence (solid triangles) and absence (hollow triangles) of thymidine.

Fig. 2 : Restriction endonuclease map of plasmid pSBT7. Streptococcal insert DNA is indicated by the heavy line. A non-complimenting derivative of pSBT7, pTTN4, was constructed by Tn 5 insertion at the position indicated by the arrow head in the above map.

Fig. 3 : Construction of the complementing plasmid pACT10. Streptococcal DNA is indicated by the heavy line.

Fig. 4 : Hybridisation of the labelled streptococcal TS gene with chromosomal DNA from S. lactis 712 and other species.

Lanes 1 and 2 refer to pBT5 and pBT2 (TS complementing cosmids) digested with Hind III. Lanes 3, 4, 5, 6 and 7 refer to Hind III cut chromosomal DNA from S. lactis 712, S. lactis MG1363 S. cremoris UC317, Lactobacillus helveticus 1829 and Pseudomonas fluorescens M114 respectively. Hybridization of the label led TS gene on a 2.7 Kb Hind III fragment to each of the DNA preparations in lanes 1-7 is shown in the corresponding lanes 1′ to 7′. The streptococcal TS gene hybridized with bands of similar size contained in Hind III digests of cosmids pBT5 (lane 1′) and pBT2 (lane 2″) and total DNA of S. lactis subsp. lactis 712 (lane 3′) and its plasmid free derivative MG1363 (lane 4′). The probe did not hybridise to total DNA of S. cremoris UC317 (lane 5′), Lactobacillus helveticus 1829 (lane 6′) or Pseudomonas fluorescens M114 (lane 7′).

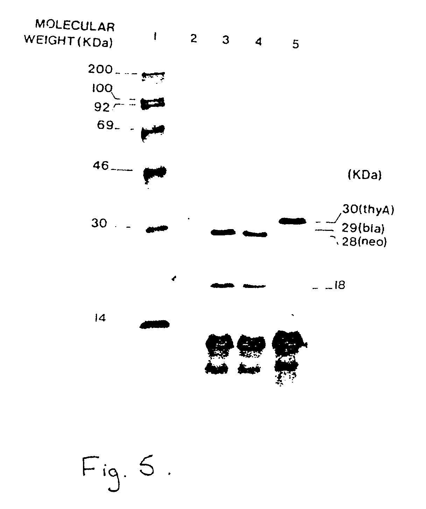

Fig. 5 : Proteins synthesised in E. coli maxicells. The fluorograph shows 355-labelled proteins produced by E. col i maxicells carrying pBR322 (lane 2), pTTN4 (lanes 3 and 4) and pSBT7 (lane 5). The 30 kDa TS sprotein is clearly identifiable in lane 5 (labelled thyA). It is likely that the protein bands at about 18 kDa in lanes 3 and 4 correspond to truncated proteins made from the TS gene disrupted by Tn5.

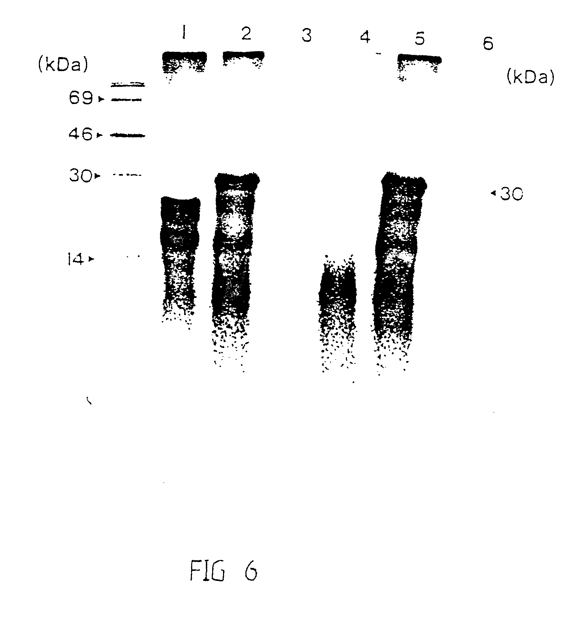

Fig. 6 : Proteins synthesised in an E. coli in vitro transcription/translation system from plasmids pACYC184 (lane 1) and pACT10 (lane 2). The TS gene of 30 kDa is clearly identifiable among the expression products of pACT10.

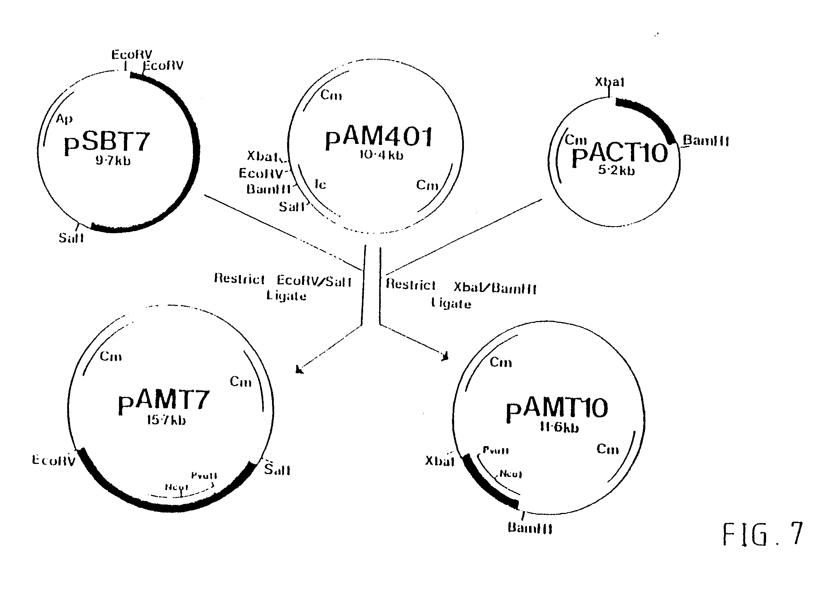

Fig. 7 : Construction of the E. coli /streptococcal shuttle plasmids pAMT7 and pAMT10. Streptococcal DNA is indicated by the heavy lines. The TS gene is on the fragment containing the Pvu II and Nco 1 sites.

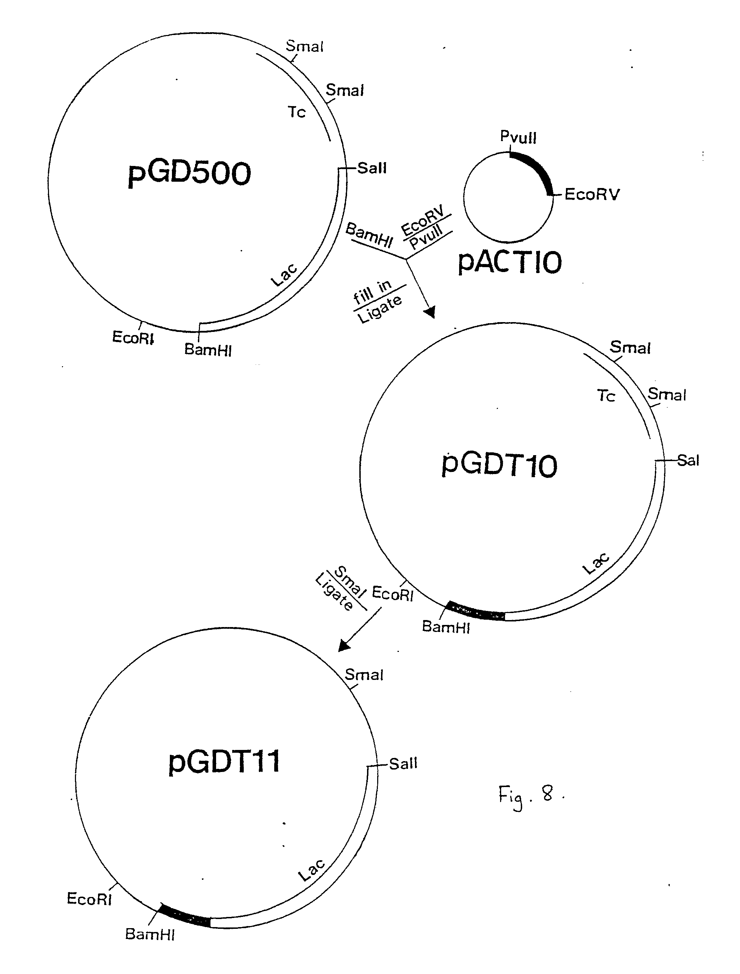

Fig. 8 : Construction of plasmid pGDT11. Streptococcal DNA is indicated by the heavy line.

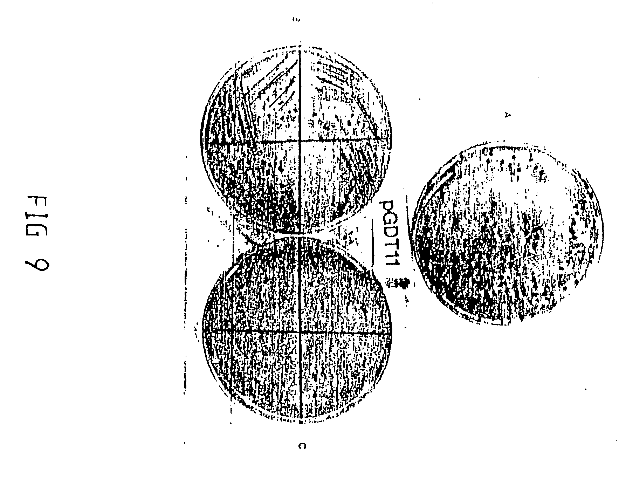

Fig. 9 : E. coli HX2 (TS⁻) transformed with pGDT11 DNA formed dark blue isolated colonies on LB-X gal (A). Four of these colonies were unable to grow on plates containing tetracycline (C) but grew well on the LB-X gal plates (B).

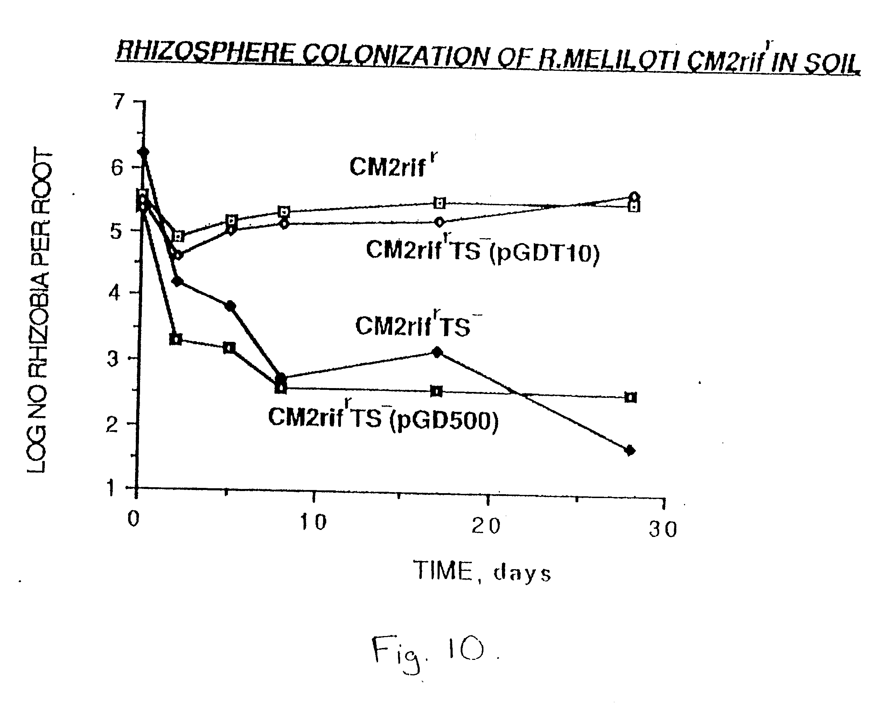

Fig. 10 : Colonization of the roots of alfalfa plants by Rhizobium meliloti CM2 Rifʳ and TS⁻ derivatives in soil. Pregerminated seeds were sterilized, coated with approximately 10⁶ cells form one of the rhizobial cultures used in the experiment and grown in non sterile soil. Plants were chosen at random and the numbers of the rhizobium strains adhering to the roots were counted on MSY plates (minimal salts with yeast extract) containing rifampicin, cycloheximide and thymidine. The TS phenotype of colonies from each strain used was confirmed.

CM2 Rifʳ : hollow squares; CM2 Rifʳ TS⁻ : solid diamonds

CM2 Rifʳ TS⁻ [pGD500] : solid squares

CMS Rifʳ TS⁻ [pGDT10] : hollow diamonds

Fig. 11 : The DNA sequence encoding the lactococcal TS gene. The predicted amino acid sequence of thymidylate synthase is shown below the condons. Both the nucleotide sequence and the amino acid sequence are numbered to the right of the sequences. Putative -10, -35 and ribosome binding sites (SD) are boxed as is a TG dinucleotide 5′ to the putative -10 sequence. The positions of the Nco I and Eco RV restriction sites are also indicated. The underlined nucleotides correspond to non-lactococcal vector DNA sequences. An inverted repeat region is indicated by arrows.

Detailed Description of the Invention

Isolation of the thymidylate synthase gene (TS) from Streptococcus lactis

A gene bank of the entire genome of Streptococcus lactis strain 712 was first constructed. A total DNA preparation extracted from S. lactis 712 was partially digested with the endonuclease Eco R1 and inserted into the Eco R1 site of DNA from the cosmid pLAFR1. The cosmids with S. lactis DNA inserts were selected following introduction into Escherichia col i LE392. The average insert size is 24 kb (SD 6.1) and

4

the total bank consists of 1,000 clones.

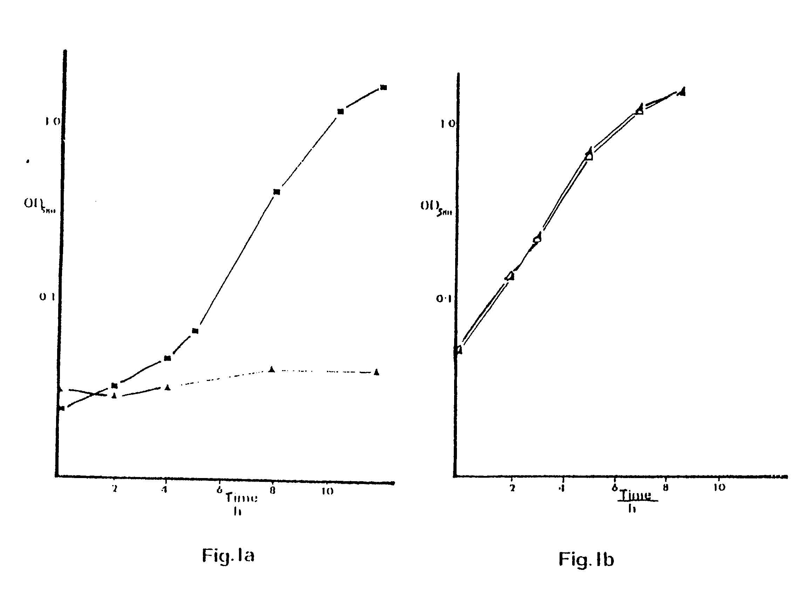

Cosmids with inserts which contained the S. lactis TS gene were identified by testing the ability of each cosmid to complement the TS⁻ mutation of E. coli X2913. Five complementing cosmid clones were identified in this way and one, pBT5, was selected for detailed analysis. Cell-free extracts of E. coli X2913 harbouring pBT5 had substantial thymidylate synthase activity whereas cell-free extracts of X2913 without the plasmid had none (Table 2). The cosmid pBT5 allowed E. coli X2913 to grow at its maximum rate in the absence or presence of thymidine (Fig. 1), indicating that the S. lactis TS gene was strongly expressed in E. coli .

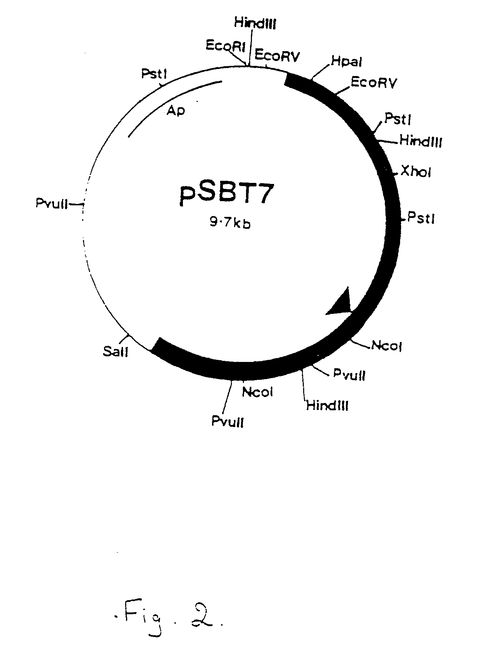

A subclone of pBT5 was constructed by partial digestion with Sau 3A into the BamH1 site of pBR322. The construct, designated PSBT7, which has 5.3 kb of S. lactis DNA, was mapped by endonuclease mapping and subjected to transposon mutagenesis by Tn5 insertion. The endonuclease site map and the position of a transposon which abolishes TS⁻ complimentation ability are shown in Fig. 2.

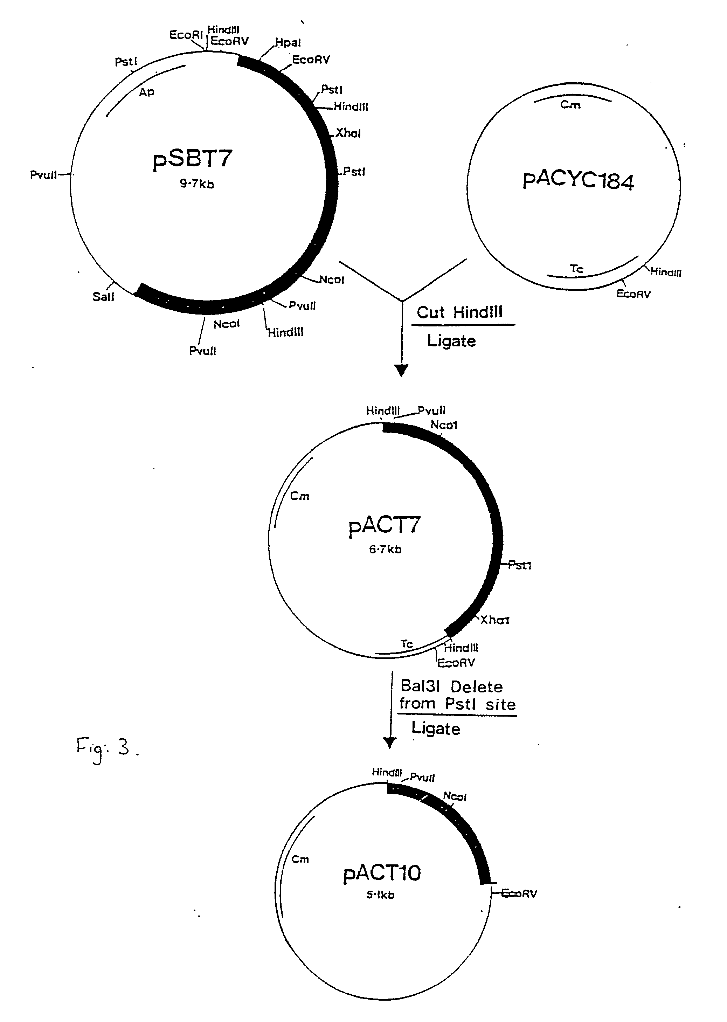

The size of the TS⁻ complementing S. lactis DNA was further reduced to about 1.2 Kb in two steps. First the 2.7 Kb internal Hind III fragment of the S. lactis insert in PSBT7 was inserted into pACYC184; the construct, pACT7 (Fig. 3) complemented TS⁻ E. coli strains. Then Bal 31 deletion from the pst I site of pACT7 was used to delete S. lactis DNA outside the TS coding area. The plasmid pACT10, with about 1.2 Kb of S. lactis DNA, had the smallest insert which complemented TS⁻ mutations in E. coli strains. The insert in pACT10 has one Nco 1 site and one Pvu II site, but the latter was subsequently shown to be outside the coding region.

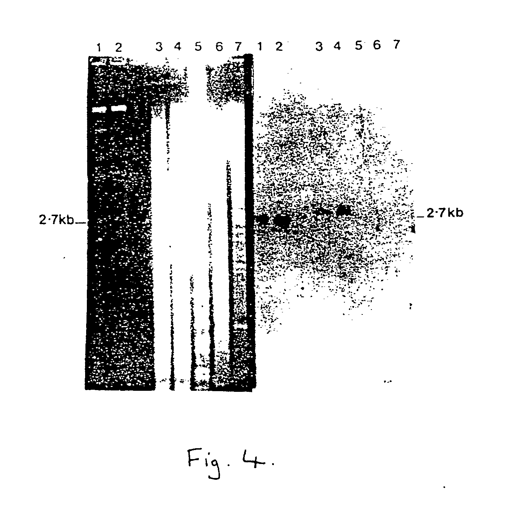

That the TS gene cloned is indeed S. lactis 712 DNA was confirmed by DNA-DNA hybridization experiments. The 2.7 Kb Hind III insert of pACT7 was labelled with biotin-11-UTP and this probe clearly hybridized with Hind III cut bands of total DNA from S. lactis 712 and its plasmid-free derivative MG1363 but not with similar preparations of DNA from S. cremoris UC317, Lactobacillus helveticus 1829 or Pseudomonas fluorescens M114 (Fig 4).

The product of the cloned S. lactis TS gene was identified as a protein of approximately 30 KDa in E. coli maxi-cell (Fig. 5) and E. coli cell-free transcription-translation preparations (Fig. 6).

The cloned S. lactis TS gene has regulatory regions which are recognised both in E. coli and S. lactis . This was shown by inserting S. lactis DNA containing the TS gene in both orientations into the shuttle vector pAM401 which replicates in E. coli and S. lactis strains ( Fig. 7). The constructs pAMT7 and pAMT10 were transformed into E. coli and S. lactis strains and expression of the TS gene from both constructs and in both bacterial species was clearly demonstrated, by assay of cell-free extracts for thymidylate synthase activity (Table 3). The fact that the cloned TS gene in pAMT7 is in the opposite orientation to that in pAMT10 and that both are equally expressed shows that the cloned fragment has its own regulatory sequences which are recognised in E. coli and S. lactis .

## Determination of the DNA sequence of the TS gene from S. lactis

The sequence of the TS gene isolated from S. lactis was derived using the chain terminating method of Sanger et al (1977) with a sequencing kit obtained from Amersham (U.K.).

## Demonstration that the Streptococcus lactis TS gene can be used as a positive selectable marker gene in transformation protocols.

In this experiment two plasmids pSBT7 (derived from pBR322) and pACT7 (derived from pACYC184) which have the S. lactis TS gene were used to transform E. coli X2913 TS⁻.

The plasmid pSBT7 consists of plasmid vector pBR322 with a 5.7 kb streptococcal insert containing the TS gene in its BamHI site. Strains containing this plasmid display a resistance to ampicillin but not tetracycline. This plasmid complements TS⁻ E. coli mutants. pSBT7 was contructed from the low copy number cosmid pBT5. This involved generating a partial Sau 3A digest of pBT5 which was ligated directly to pBR322 previously digested with BamHI. The resultant plasmid DNA was transformed into E. coli X2913 TS⁻ using ampicillin resistance (Apʳ) of pBR322 as a selectable marker. From the Apʳ transformants, TS⁺ clones were identified by patching on LB plates since E. coli X2913 does not grow on LB without supplementation with thymidine. Approximately 2% of the resultant clones carried the TS gene. Plasmid DNA isolated from these complementing clones varied in size from 9.7 kb - 17 kb. The smallest of these plasmids pSBT7 was selected for further study.

The plasmid pACT7 consists of plasmid vector pACYC184 with a 2.7 kb streptococcal insert containing the TS gene. Strains containing this plasmid display a resistance to chloramphenicol but not tetracycline. This plasmid also complements 75 E. coli mutants. pAT7 was contructed simply by ligating HindIII cut pSBT7 with HindIII cut pACYC184. Resultant plasmid DNA was transformed into E. coli HX2 TS⁻ using chloramphenicol resistance (Cmʳ) of pACYCl84 as a selectable marker. All the resultant complementing plasmids contained the 2.7 kb HindIII fragment. One such plasmid was designated pACT7.

Approximately 1μg of plasmid DNA and $10^{10}$ competent recipient cells were used in each transformation. Each transformation mix was plated on LB plates which do not have sufficient thymidine to support growth of E. coli X2913 TS⁻. Over $10^4$ colonies grew from each transformation mix. Control plates with X2913 alone showed no growth.

Since pSBT7 has the ampicillin resistance gene of pBR322 and, likewise, PACT7 the chloramphenicol resistance gene of pACYC184, 100 TS⁺ colonies from each transformation were tested for acquisition of the relevant antibiotic resistance marker. All were positive, proving that the TS gene was an efficient marker gene of pSBT7 and pACT7 and could be used as the primary positive selection marker in transformation experiments.

## Comparison of the efficiency of the Streptococcus lactis TS gene with that of antibiotic resistance genes as selectable markers in transformation experiments

In this experiment two plasmids, pBRT10 which has the cloned TS gene and an Apʳ gene and pACT10 which has the TS gene and a Cmʳ gene were used to transform E. coli HX2 a TS⁻ derivative of E. coli HB101 which is ampicillin sensitive and chloramphenicol sensitive. About 8 x $10^{10}$ HX2 cells and 0.5μg of plasmid DNA were used in each transformation. Plasmid DNA from pACYC184 from which pACT10 was constructed was used as a control.

The transformation mixes were plated on LB plates (selects TS⁺ transformants), LB ampicillin and LB chloramphenicol plates. The results in Table 4 show that the numbers of transformants were similar whether the genetic marker used for selection was TS, or antibiotic resistance.

One hundred TS⁺ transformant colonies from each experiment were checked for the acquisition of the antibiotic resistance gene of the plasmid used in the transformation; all had acquired the relevant marker. The experiment clearly shows that the TS gene is as efficient a selection marker as the ampicillin resistance or chloramphenicol resistance genes of plasmids derived from pBR322 or pACYC184.

## Construction of a cloning vector which relies on the thymidylate synthase gene of Streptococcus lactis 712, instead of antibiotic resistance markers, as a positive selection marker.

Plasmids designed for genetic engineering of strains ultimately to be released into the food chain or the environment, should be free of antibiotic resistance genes. We have constructed a plasmid pGDT11 which has the S. lactis TS gene fused to the E. coli β-galactosidase gene, is free of antibiotic resistance genes and has suitable sites available for cloning of other genes.

Plasmid pGDT11 was derived from pGD500 a broad host range Gram negative plasmid with a tetracycline resistance (Tetʳ) gene and promoterless lac operon downstream of a Bam H1 cloning site (Fig. 8). The TS gene of pACT10 was inserted into this Bam H1 site. The resultant construct, pGDT10, was obtained by transformation of E. coli HX2 (TS⁻) and selection on LB plates with tetracycline. When PGDT10 was retransformed into E. coli 7118 lac and plated on LB tetracycline plates with X-Gal (5-bromo-4-chloro-3-indolyl-β-D-galacto-pyranosidase), all the transformants were dark blue, showing that in pGDT10 the TS gene was fused to the lac operon, such that the lac operon DNA was being expressed from a promoter on the DNA fragment encoding the TS gene.

To complete the construction, the tetʳ gene was excised by digestion of pGDT10 with Sma 1 and religation; the construct, pGDT11, was obtained by transformation of E. coli HX2 (TS⁻) and selection on LB plates with X-Gal but without tetracycline. The transformant colonies were blue and sensitive to tetracycline (Fig. 9). pGDT11 has several sites (e.g. Bam H1, Bgl II, Sal 1) which can be used for cloning other genes. In addition, the TS-lac fusion can be excised from pGDT11 by cutting with Bam H1 and Sal 1 and used to construct other plasmids without recourse to antibiotic selection. The TS-lac fusion has an Eco R1 site in the lac Z gene which also can be used for cloning, and clones with inserts will be distinguishable on LB-XGal plates as white colonies due to insertional inactivation of the lac Z gene.

The plasmid pGDT11 was deposited in E. coli HX2 at the National Collection of Industrial and Marine Bacteria, Torry Research Station, Aberdeen, Scotland on 2nd June 1989 under accession No. 40153.

Demonstration that the streptococcal TS gene can be used as a positive selectable marker in bacteria other than Escherichia coli

Construction of pGDT10 (Fig. 8) derived from the broad Gram negative host range plasmid pGD500 allowed the efficiency of the TS gene as a selectable gene marker in a Gram negative bacterium other than E. coli to be tested. The agronomically important Rhizobium meliloti CM2 was chosen as the test bacterium and from this strain TS⁻ mutants were isolated by plating out approximately 5 X 10⁹ cells on MSG medium (minimal salts glutamate medium, O'Gara and Shanmugan, 1976) containing thymidine and the anti-folate drug aminopterin according to the method of Stacey and Simpson (1965).

One of these mutants R. meliloti CM21 was used to compare the efficiencies of the TS gene and the tetracycline resistance (Tcʳ) gene of pGDT10 to select CM21 strains carrying the plasmid. Using the plasmid pRK2013 as a helper plasmid pGDT10 was conjugated into R. meliloti cm2 accoridng to the method of Ditta et al . (1980) and the mating mix was plated on MSG and MSG containing both tetracycline and thymidine. The numbers of pGDT10 transconjugant colonies were similar on both media (7 x 10⁻⁴ per recipient). In control mating pGD500 transconjugant colonies appeared on MSG plates containing tetracycline and thymidine but not on MSG plates alone. These results show that the streptococcal TS gene complemented the TS mutation of R. meliloti CM21 and further demonstrated that the TS gene was just as efficient a primary selection marker as the tetracycline resistance gene in conjugation experiments.

Dependence of a TS⁻ mutant of Rhizobium meliloti on the streptococcal TS gene for colonisation of alfalfa plants

A TS⁻ mutant of a rifampicin resistant (Rifʳ) derivative of R. meliloti CM2 was made as indicated in the previous section. In addition the pGDT10 plasmid containing the TS gene from S. lactis and pGD500, from which pGDT10 was derived, were conjugated into the R. meliloti Rifʳ TS⁻ cultures. The abilities of R. meliloti CM2 Rifʳ, R. meliloti Rifʳ TS⁻, R. meliloti Rifʳ TS⁻ [pGD500] and R. meliloti Rifʳ TS⁻ [pGDT10] to colonize alfalfa plants was measured. Pre germinated alfalfa seeds were inoculated with each of the 4 strains grown in soil and analysed for rhizobial numbers according to the method of Stephens et al (1987). The results (Fig. 10) show that pGDT10 allows the TS⁻ mutant of R. meliloti to survive as well as the wild type TS⁺ strain whereas the numbers of the TS⁻ mutant and the TS⁻ mutant containing the control plasmid pGD500 decreased over 1,000 fold in 30 days.

Furthermore, nodules were formed on all 20 plants inoculated with R. meliloti Rifʳ TS⁻ containing pGDT10 whereas nodules were not formed on any plants inoculated with the TS⁻ mutant or the TS⁻ mutant containing pGD500. This experiment clearly shows that the TS gene from Streptococcus lactis allows stable maintenance of the plasmid pGDT10 in a natural environment.

## Abbreviations

| | |
|---|---|
| GEMS | genetically engineered microbes |
| TS | thymidylate synthase |
| G.R.A.S. | generally recognised as safe |
| LB | Luria and Bertani (medium) |
| X-gal | 5-bromo-4-chloro-3-indolyl-B-D-galacto-pyranosidase |
| Apʳ | ampicillin resistant |
| Cmʳ | chloramphenicol resistant |
| tetʳ | tetracycline resistant |
| Rifʳ | rifampicin resistant |
| MSG | minimal salts glutamate medium |
| MSY | minimal salts with yeast extract |

N.B.

Streptococcus lactis has been renamed Lactococcus lactis subsp. lactis but the former name has been used in this text.

REFERENCES

Herman R.E. and McKay L.L. (1986) Appl. Environ. Microbiol. 52, 45-50

de Vos W.M. (1986) Neth. Milk Dairy J. 40, 141-154.

de Vos W.M. (1987) Ferns Microbiol. Rev. 46, 281-295.

de Vos and Simons (1988) Biochemie 70, 461-473.

Hardy L.W., Finer-Moore J.S., Monfort W.R., Jones M.O., Santi D.V. and Stroud R.M. (1987) Science 228, 448-455.

Maniatis T., Fritsch E.F. and Sambrook J. (1982) Molecular Cloning - A laboratory manual, Cold Spring Harbor Laboratory, Cold Spring Harbor, NY.

Belfort M., Maley G., Pederson-Lane J. and Maley F. (1983) Proc. Natl. Acad. Sci. USA 80, 4914-4918.

Pinter K., Davisson V.J. and Santi D.V. (1988) DNA 7, 235-241.

Chu F.K., Maley G.F., Maley F. and Belfort M. (1984) Proc. Natl. Acad. Sci. USA 81, 3049-3053.

Kenny E., Atkinson T. and Hartley B.S. (1985) Gene 34, 335-342.

Honess R.W., Bodemer W., Cameron K.R., Niller H.N., Fleckenstein B. and Randall R.E. (1986) Proc. Natl. Acad. Sci. USA 83, 3604-3608.

Thompson R., Honess R.W., Taylor L., Morran J. and Davison A.J. (1987) J. Gen. Virol. 68, 1449-1455.

Bzik D.J., Toshihiro W.L. and Inselburg J. (1987) Proc. Natl. Acad. Sci. USA 84, 8360-8364.

Beverley S.M., Ellenberger T.E. & Cordingley J.S. (1986) Proc. Natl. Acad. Sci. USA 83, 2584-2588.

Takeishi K., Kaneda S., Ayusawa D., Simizu K., Goboh O., and Seno T. (1985) Nucleic Acids Res. 13, 2035-2043.

Perryman S.M., Rossana C., Deng T., Vanin E.F., & Johnson L.F. (1986) Mol. Biol. Evol. 3, 313-321.

0'Gara, F. and Shanmugam, K.T. 1976. Biochem. Biophys. Acta. 437 : 313-321.

Stacey, K.A. and Simson, E. 1965. Bacteriol. Proc. 90 : 554-555.

Ditta, J.T., Stanfield, S., Corbin, D. and Helinski, D.R. 1980. Proc. Natl. Acad. Sci. USA. 77: 7347-7351.

Stephens, P.M., O'Sullivan, M. and O'Gara, F. 1987. Applied and Environmental Microbiology 53 : 1164-1167.

Sanger, F., Nicklen, S. and Coulson, A.R. 1977. Proc. Natl. Acad. Sci. 74 : 5463-5467.

Table 1

| Sources of cloned and sequenced thymidylate synthase gene | |
|---|---|
| Source | Reference |
| Escherichia coli | Belfort et al ., 1983 |
| Lactobacillus casei | Pinter et al ., 1988 |
| T4 phage | Chu et al ., 1984 |
| Q 3T phage | Kenny et al ., 1985 |
| Herpesvirus saimiri | Honess et al ., 1986 |
| Varicella zoster virus | Thompson et al ., 1987 |
| Plasmodium falciparum | Bzik et al ., 1987 |
| Leishmania major | Beverley et al ., 1986 |
| Human cells | Takeishi et al ., 1985 |
| Mouse cells | Perryman et al ., 1986 |

TABLE 2

| Thymidylate synthase activity in Escherichia coli X2913 (TS⁻) cells complemented by plasmids with inserts of DNA of Streptococcus lactis 712. | |
|---|---|
| Plasmid Strain | Thymidylate Synthase Activity ($\mu$mol.min-1.mg protein) |
| 1. pLAFR1 | 0[a] |
| 2. pBT5[b] | 0.043 ± 0.011 |
| 3. pBT5[b] | 0.05 ± 0.013[a] |

[a]Thymidine (50$\mu$g/ml) added to growth medium.
[b]pLAFRI with a 22 Kb Streptococcus lactis 712 insert.

TABLE 3

| Thymidylate synthase activity in cells containing pAMT10 and pAMT7 plasmids. | |
|---|---|
| Plasmid Strain | Thymidylate Synthase Activity ($\mu$mol.min⁻¹.mg protein) |
| E. coli HX2 [pAMT7] | 0.047 ± 0.006 |
| E. coli HX2 [pAMT10] | 0.024 ± 0.008 |
| E. coli HX2 [pAM401] | 0 |
| S. lactis MG1363 [pAMT7] | 0.035 ± 0.002 |
| S. lactis MG1363 [pAMT10] | 0.032 ± 0.005 |
| S. lactis MG1363 | $2.48 \times 10^{-3} \pm 1.54 \times 10^{-3}$ |

TABLE 4

| Plasmid | Number of transformants (units of $10^5/\mu$g DNA) using as selection marker | | |
|---|---|---|---|
| | TS⁺ | Ap[r] | Cm[r] |
| pBRT10 | 0.97 | 0.8 | - |
| pACT10 | 8.6 | - | 7.1 |
| pACYC184 | 0 | - | 6.8 |

**Claims**

1. A vector devoid of antibiotic resistance genes and bearing a thymidylate synthase gene as a selection marker.

2. A vector as claimed in claim 1 wherein the thymidylate synthase gene is derived from a G.R.A.S. (generally recognised as safe) micro-organism.

3. A vector as claimed in claim 1 or claim 2 wherein the thymidylate synthase gene is derived from Lactobacillus casei .

4. A vector as claimed in claim 1 or claim 2 wherein the thymidylate synthase gene is derived from Streptococcus lactis .

5. A vector as claimed in any preceding claim wherein the thyrnidylate synthase gene is the gene deposited in plasmid pGDT11 at the National Collection of Industrial and Marine Bacteria, Torry Research Station, Aberdeen, Scotland, under accession No. 40153, or a gene substantially similar thereto, the said gene encoding thymidylate synthase activity.

6. A vector as claimed in claim 4 wherein the thymidylate synthase gene has the DNA sequence shown in Figure 11, or a sequence substantially similar thereto, the said sequence encoding thymidylate synthase activity.

7. The thymidylate synthase gene deposited at the National Collection of Industrial and Marine Bacteria, Torry Research Station, Aberdeen, Scotland, under accession No. 40153, or a gene which is substantially similar thereto, the said gene encoding thymidylate synthase activity.

8. A thmidylate synthase gene having the DNA sequence shown in Figure 11, or a sequence substantially similar thereto, the said sequence encoding thymidylate synthyase activity.

9. A thymidylate synthase gene as claimed in claim 7 or claim 8 in biologically pure form.

10. Host cells comprising a vector as claimed in any of claims 1 to 6 or a gene as claimed in any of claims 7 to 9.

11. Host cells as claimed in claim 10 in biologically pure form.

12. A method of genetically labelling a cell which has received a gene of interest comprising:-

(a) coupling the gene of interest with a thyrnidylate synthase gene,

(b) transforming a recipient cell, which is devoid of thyrnidylate synthase activity, with the coupled gene of interest and thymidylate synthase gene,

such that cells which have received the gene of interest will be thymidylate synthase positive.

13. A method of stably maintaining a gene of interest in a cell which has received the gene of interest by genetic manipulation, comprising:-

(a) coupling the gene of interest to a thymidylate synthase gene,

(b) transforming a recipient cell, which is devoid of thymidylate synthase activity, with the coupled gene of interest and thymidylate synthase gene, and

(c) maintaining the recipient cell in a culture medium devoid of or limited in thymidine or thymine, such that only cells which maintain the thymidylate synthase gene grow.

Fig.1b

Fig.1a

.Fig . 2 .

Fig. 3.

Fig. 4.

MOLECULAR
WEIGHT (KDa)

Fig. 5.

FIG 6

FIG. 7

EP 0 406 003 A1

Fig. 8.

pGDT11

FIG 9

RHIZOSPHERE COLONIZATION OF R.MELILOTI CM2rif$^r$ IN SOIL

Fig. 10.

```
                                          -35                        -10
TCT GAG AGG TTA TTT TGG GAA ATA CTA|TTG AAC|CAT ATC GAG GTG GTG|TGG|TAT AAT|GAA 60

                    SD
. GGG AAT TAA AAA AGA TAG GAA AAT TTC ATG ACT TAC GCA GAT CAA GTT TTT AAA CAA AAT 120
                                        Met Thr Tyr Ala Asp Gln Val Phe Lys Gln Asn

ATC CAA AAT ATC CTA GAT AAT GGT GTT TTT TCA GAA AAT GCA AGA CCA AAG TAT AAG GAT 180
Ile Gln Asn Ile Leu Asp Asn Gly Val Phe Ser Glu Asn Ala Arg Pro Lys Tyr Lys Asp

GGT CAA ATG GCG AAT AGC AAA TAT GTC ACT GGT TCA TTC GTT ACT TAT GAT TTG CAA AAG 240
Gly Gln Met Ala Asn Ser Lys Tyr Val Thr Gly Ser Phe Val Thr Tyr Asp Leu Gln Lys

GGG GAG TTT CCA ATT ACC ACT TTG CGT CCA ATT CCA ATC AAA TCT GCT ATT AAA GAA TTG 300
Gly Glu Phe Pro Ile Thr Thr Leu Arg Pro Ile Pro Ile Lys Ser Ala Ile Lys Glu Leu

ATG TGG ATA TAC CAA GAC CAA ACA AGT GAA CTT TCT GTT CTC GAA GAG AAG TAT GGA GTC 360
Met Trp Ile Tyr Gln Asp Gln Thr Ser Glu Leu Ser Val Leu Glu Glu Lys Tyr Gly Val

AAA TAC TGG GGA GAA TGG GGA ATT GGT GAT GGT ACG ATT GGG CAA CGT TAT GGT GCA ACA 420
Lys Tyr Trp Gly Glu Trp Gly Ile Gly Asp Gly Thr Ile Gly Gln Arg Tyr Gly Ala Thr

                                                                   NcoI
GTC AAA AAA TAT AAT ATC ATT GGT AAA TTA TTA GAA GGC TTG GCC AAA AAT CCA TGG AAT 480
Val Lys Lys Tyr Asn Ile Ile Gly Lys Leu Leu Glu Gly Leu Ala Lys Asn Pro Trp Asn

CGT CGT AAT ATC ATC AAC CTT TGG CAG TAT GAA GAT TTT GAG GAA ACA GAA GGT CTT TTA 540
Arg Arg Asn Ile Ile Asn Leu Trp Gln Tyr Glu Asp Phe Glu Glu Thr Glu Gly Leu Leu

CCA TGT GCT TTC CAA ACG ATG TTT GAT GTC CGT CGA GAA AAA GAT GGT CAG ATT TAT TTG 600
Pro Cys Ala Phe Gln Thr Met Phe Asp Val Arg Arg Glu Lys Asp Gly Gln Ile Tyr Leu

GAT GCC ACA CTG ATT CAA CGT TCA AAC GAT ATG CTT GTA GCC CAC CAT ATC AAT GCG ATG 660
Asp Ala Thr Leu Ile Gln Arg Ser Asn Asp Met Leu Val Ala His His Ile Asn Ala Met

CAA TAT GTT GCT TTG CAA ATG ATG ATT GCA AAA CAT TTT TCT TGG AAA GTT GGG AAA TTC 720
Gln Tyr Val Ala Leu Gln Met Met Ile Ala Lys His Phe Ser Trp Lys Val Gly Lys Phe

TTT TAT TTT GTA AAT AAT TTA CAT ATT TAT GAT AAT CAG TTT GAG CAG GCA AAT GAA TTA 780
Phe Tyr Phe Val Asn Asn Leu His Ile Tyr Asp Asn Gln Phe Glu Gln Ala Asn Glu Leu

ATG AAG CGA ACA GCT TCT GAA AAA GAA CCT CGT TTG GTC CTT AAT GTT CCT GAT GGT ACA 840
Met Lys Arg Thr Ala Ser Glu Lys Glu Pro Arg Leu Val Leu Asn Val Pro Asp Gly Thr

AAC TTT TTC GAT ATT AAA CCT GAA GAT TTT GAA CTT GTG GAC TAT GAG CCA GTA AAA CCT 900
Asn Phe Phe Asp Ile Lys Pro Glu Asp Phe Glu Leu Val Asp Tyr Glu Pro Val Lys Pro

                                                          ───────▶   ◀───────
CAA TTG AAA TTT GAT TTA GCA ATT TAA ATT AAT CTA TAA GTT ACT GAC AAA ACT GTC AGC 960
Gln Leu Lys Phe Asp Leu Ala Ile End

ACC GTC ACC CTG GAT GCT GTA GGC ATA GGC TTG GTT ATG CCG GTA CTG CCG GGC CTC TTG 1020

    EcoRV
CGG GAT ATC
```

Fig. 11.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-251579 (ENTEROVAX RESEARCH PTY. LTD.)<br>* column 1, line 38 - column 3, line 5 *<br>* column 5, lines 7 - 29; claims 1, 4, 7, 13, 17 * | 1-2, 6, 8-13 | C12N15/52<br>C12N15/54<br>C12N1/21 |
| Y | | 3, 5, 7 | |
| D,Y | DNA<br>vol. 7, no. 4, 1988, Mary Ann Liebert, Inc.<br>pages 235 - 241; PINTER, K. et al.:<br>"Cloning, sequencing, and expression of the Lactobacillus casei Thymidylate synthase gene"<br>* the whole document * | 3, 5, 7 | |
| A | PROCEEDINGS OF THE NATIONAL ACADEMY OF SCIENCES OF USA.<br>vol. 84, no. 23, December 1987, WASHINGTON US<br>pages 8360 - 8364; BZIK, D.J. et al.:<br>"Molecular cloning and sequence analysis of the Plasmodium falciparum dihydrofolate reductase-thymidylate synthase gene"<br>* the whole document * | 3-11 | |
| T | APPLIED AND ENVIRONMENTAL MICROBIOLOGY<br>vol. 56, no. 7, July 1990, American Society for Microbiology<br>pages 2156 - 2163; ROSS, P. et al.:<br>"Cloning and characterization of the thymidylate synthase gene from Lactococcus lactis subsp. lactis."<br>* the whole document * | 1-13 | TECHNICAL FIELDS SEARCHED (Int. Cl.5 )<br><br>C12N |
| T | APPLIED AND ENVIRONMENTAL MICROBIOLOGY<br>vol. 56, no. 7, July 1990, American Society for Microbiology<br>pages 2164 - 2169; ROSS, P. et al.:<br>"Thymidylate synthase gene from Lactococcus lactis as a genetic marker: an alternative to antibiotic resistance genes."<br>* the whole document * | 1-13 | |

The present search report has been drawn up for all claims

| Place of search | Date of completion of the search | Examiner |
|---|---|---|
| THE HAGUE | 05 SEPTEMBER 1990 | ANDRES S.M. |